# EUROPEAN PATENT SPECIFICATION

(11) **EP 4 213 789 B1**
(45) Date of publication and mention of the grant of the patent: **21.08.2024**
(21) Application number: 21810089.9
(22) Date of filing: 17.09.2021
(51) Int. Cl.: A61G 13/00, A61H 1/02, A61H 3/00

(54) **VERTICALIZING TABLE EQUIPPED WITH MEANS OF MOBILIZATION OF THE LOWER LIMBS AND LIGHT SOURCES**
VERTIKALISIERTISCH MIT MOBILISIERUNG DER UNTEREN GLIEDMASSEN UND LICHTQUELLEN
TABLE DE VERTICALISATION ÉQUIPÉE DE MOYENS DE MOBILISATION DES MEMBRES INFÉRIEURS ET SOURCES LUMINEUSES

(30) Priority: 21.09.2020 IT 202000022195
(43) Date of publication of application: 26.07.2023
(73) Proprietor: Pandhora S.r.l., 80133 Napoli (NA) (IT)
(72) Inventor: TRONCONE, Stefano, 84129 Salerno (SA) (IT)
(74) Representative: Conversano, Gabriele
(86) International application number: PCT/IB2021/058502
(87) International publication number: WO 2022/058956

(56) References cited:
- EP-A1- 1 169 003
- WO-A1-2012/148064
- WO-A1-2020/250092
- US-A- 6 024 760
- US-A1- 2009 054 217

## Description

The present invention relates to a robot equipment for rehabilitation. In particular, the present invention relates to a verticalization table provided with lower limbs mobilization means and sources of infrared radiation.

Robotic rehabilitation allows to regain an almost normal life even after traumas and invalidating pathologies. In fact, the usage of robots for physiotherapy has opened up new horizons in the treatment of permanent and temporary disability. These are in fact robotic systems for limbs re-education.

In particular, devices exist for robotic rehabilitation made up of verticalization tables, which can be moved from a horizontal position to a vertical position, on which a patient is laid and fastened by suitable harnesses. Then the table the patient is laid on is brought gradually from the horizontal to the vertical position, and dedicated kinematisms make the patient carry out movements with the lower limbs.

Verticalization tables provided with robotized steppers are known, as for example the ones described in Patents EP2730265 and EP1169003. The functioning is the following: when the patient is verticalized the steppers provide a mobilization of the lower limbs which can occur both actively (i.e. with the patient participating to the muscle effort) and passively.

The machine moves the lower limbs with a programmed speed according to a scheme of alternated flexions and extensions, thus stimulating the active participation of the patient according to his capacity of collaboration.

Generally, in all the known devices the upper portion of the body is held to the tilting table by means of a harness fastening the patient at his chest and shoulders.

The thighs of the patient are fixed to suitable movement mechanisms and the feet are fastened to suitable movable plates provided with springs. Consequently, the flexion and extension movements of the hips caused by the movement mechanisms impose also a loading and discharging action of the bearing force on the feet. In this way, the therapy influences the movements of the joints of hips, knees and ankles. The mobilization sequence of the lower limbs is controlled by a computer which allows to adjust the movement of the legs, the load, the speed, the extension of the hip and other parameters. The advantage of these devices is that they allow to carry out a fast and intensive therapy.

In order to increase the proprioceptive stimulation of the patient (i.e. his awareness of the movement he is carrying out) in the devices known at the state of the art monitors are used, positioned so that the patient can see them, which reproduce as virtual reality the movements he is carrying out.

In this way, the therapy becomes even more effective since the patient is able to associate mentally the movement he is carrying out to the sensations of his own legs.

Anyway, the perception of the patient of the movement he is carrying out is not of physical type but exclusively visual. Moreover, verticalization tables exist provided with robotized steppers and devices configured to apply electrical stimulations to the patient according to a scheme associated to the movement he is carrying out, in order to maximize the therapy by acting in this manner on the neuronal plasticity. These systems apply the integrated functional electric stimulation (FES) which increases the effects of the early verticalization on the cardiovascular system.

Anyway, this kind of stimulation is very invasive, since it needs electrodes to be installed on the patient's legs, which make electric current pass through the muscle in order to stimulate it. For many subjects, for example children, the electric stimulation can be uncomfortable. An electric stimulation of the leg muscles can be not tolerated.

At the best of the current inventors' knowledge there are not known devices of the just described type which implement technical solutions intended to optimize the effectiveness of the rehabilitation treatment, and in particular to improve the physical perception of the movement the user is carrying out.

In other technical fields, there are also known systems combining the infrared radiation with muscle movements, for training or for improving the loosing of weight in determined body parts. US6024760 describes a device to reduce adipose tissue in determined areas by stimulating lipolysis through the heat provided by generators of infrared rays while the user carries out fitness exercises or general muscle activity.

WO212148064A1 describes a fitness tool for abdominal friction exercises for abdominal loosing of weight comprising an infrared lamp directed to the portion of the abdomen object of friction. US2009054217 describes a tiltable inversion fitness tool which comprises a generator of infrared rays to facilitate or favour blood circulation.

So, there are generally known techniques providing the usage of infrared rays to improve blood circulation and for muscle relaxation, but there are not known techniques where the infrared stimulation is used to improve the perception a user has of his own body and movements he is carrying out.

Therefore, aim of the present invention is to provide a verticalization table which overcomes the limits linked to the embodiments known at the state of the art, and which optimizes the effectiveness of the treatment and the perception each user has of the movement he is carrying out.

According to an aim the present invention is intended to facilitate the post-traumatic rehabilitation by using infrared radiations combined with the muscle movement obtained by means of a verticalization table.

The present invention realizes the prefixed aims since it is a device for mobility rehabilitation comprising a table (2), hinged at an end to a support structure (3) and respective actuator (4), configured to make said table (2) rotate between a horizontal position and a vertical position, said table (2) being provided with a harness (15) configured to fasten a patient positioned thereon so that the patient cannot fall down even when the table is in vertical position, said rehabilitation device comprising also a lower limbs movement mechanism (8) configured to make the patient's femur rotate with respect to the hip and a couple of feet movement platforms (6), characterized in that said device comprises also a plurality of infrared light sources (11), fastened to respective supports (10) integral to said support structure (3) and positioned so that each light source lights up the muscles of a respective area of the patient's lower limbs, a data processing and control unit (13) configured to control said lower limbs movement mechanism (8) and said light sources (11) and in that said data processing and control unit (13) is configured also to manage an adjusting cycle of the intensity of said light sources (11) in a differentiated manner for each of said sources, as a function of the movement cycle imposed by said couple of mobilization actuators of the lower limbs.

The present invention is described in the following with reference to the appended figures:

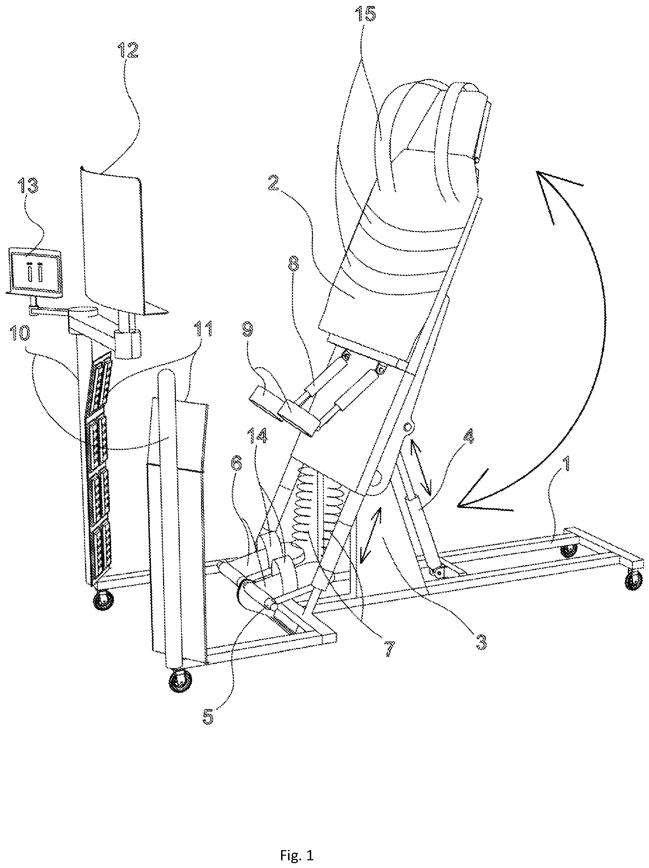

Fig. 1 shows a perspective view of the invention with all its essential elements and in which the arrows indicate the possible movements of some components of the device.
Fig. 2 shows a side view of the invention illustrating the table (2) in almost vertical position with the data processing and control unit (13) managing an adjusting cycle of the intensity of said light sources (11), in a differentiated manner for each of said sources, as a function of the movement cycle imposed by said couple of lower limbs mobilization actuators (8).

The arrows indicate the movement induced by the lower limbs mobilization actuators (8).

Fig. 3 shows a front view of the invention in which the light sources (11) irradiate only the left leg of the patient corresponding at the same time in which it is moved by the left actuator.

Fig. 4 shows a side perspective view of an application of the invention illustrating the functioning of the invention in the configuration with verticalization table (2) in horizontal position.

Fig. 5 shows a schematic indication of the light intensity variation cycles.

With reference to what shown in the appended figures, the device according to the invention comprises a table (2), hinged at an end to a support structure (3) integral to a base (1) on rollers and a respective actuator (4), configured to make said table (2) rotate between a horizontal position and a vertical position, said table being configured to take stably any position between the horizontal and the vertical one.

In a preferred and not limiting embodiment, said actuator (4) is a hydraulic or electric piston hinged at an end to the base (1), and at the other end to the table (2).

The table (2) is also provided with a harness (15) configured to fasten the patient's chest and shoulders so that the patient cannot fall down even when the table is in vertical position.

The device comprises also a lower limbs movement mechanism (8).

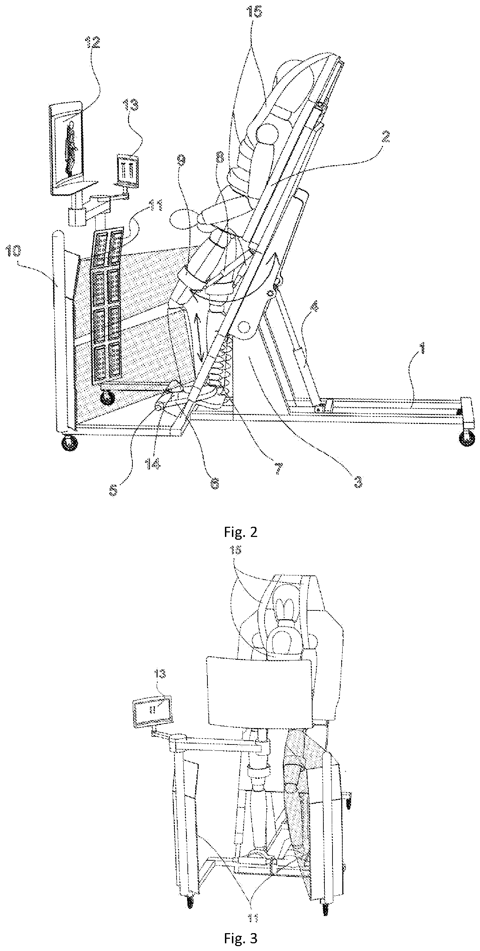

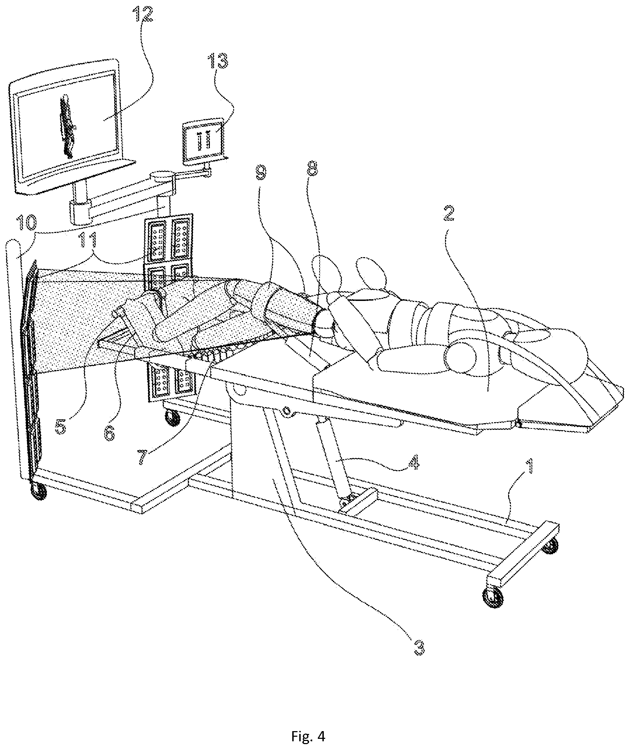

In an embodiment, said mechanism comprises a couple of linear actuators, of electric or hydraulic type, each hinged at an end to the table (2) at the position of the hip of each leg of the patient and at the opposite end to a respective fastening means (9) for the patient's femur. Preferably, the fastening means (9) comprises a semicircular rigid support inside which the patient's femur can be fastened by means of a band, when the patient lays on the verticalization table (2). From the just described geometry it is clear that according to what shown for example in figures 2 and 4, a rotation movement of the patient's femur with respect to the hip corresponds to an extension of each one of said actuators (8).

Generally, the table comprises a kinematism and respective movement means configured to make the patient's femur rotate with respect to the hip, so that it is possible to control the movements of the lower limbs of the patient.

The device comprises also a couple of feet movement platforms (6) positioned at the position of the patient's feet, each hinged with its own front end to an axis rotation (5).

In a first embodiment said axis of rotation (5) is integral to the verticalization table (2); in a second embodiment said axis is fastened so that it can translate according to a parallel direction to said table (2), so that the foot can carry out a roto-translation movement as a whole.

Each platform (6) is also provided with a spring (7) or other elastic pushing means configured to push upwards the rear portion of said platform (6) making it rotate around its own axis of rotation.

It is clear that an elongation or compression of the respective pushing means corresponds to a rotation of each platform (6) around its own axis of rotation positioned at the front end.

The springs (7) allow the platforms (6) to support the foot during the execution of the whole movement of the leg induced by the actuator (8).

The device comprises also a plurality of infrared light sources (11), fastened to respective supports (10) integral to the base (1), and positioned so that each light source lights up the muscle of a respective area of the patient's lower limbs, and a data processing and control unit (13) provided with a display (12) positioned so that the user can see it and configured to control said lower limbs movement mechanism (8) and said light sources (11). The device is characterized in that said data processing and control unit (13) is configured to manage an adjusting cycle of the intensity of said light sources (11), in a differentiated manner for each of said sources, as a function of the movement cycle imposed by said couple of lower limbs mobilization actuators.

In a preferred embodiment, on said processing and control unit (13) computer programs are stored configured to implement the following method:
1) subdividing the lower limbs in a plurality of areas, each area being associated to the stimulation by means of a respective infrared light source (11);
2) for each of said areas defined at point 1, identifying a main muscle group and schematizing the action cycle imposed by said couple of lower limbs mobilization actuators (8) as a sequence of a plurality of contraction and relaxation steps of said main muscle group;
3) determining, for each of said light sources (11) associated to said areas, an adjusting cycle of the intensity of the infrared radiation emitted as a function of said contraction and relaxation cycle schematized at point (2) for the area associated to the specific light source.

The adjusting cycle of the intensity is preferably configured so that each light source has a maximum emission intensity at the contraction steps of the respective muscle group and a minimum emission intensity at the relaxation steps of the respective muscle group.

Each light source can be possibly switched on with a predetermined advance of time with respect to the contraction step of the respective muscle group.

In another embodiment the device is characterized in that the movement mechanism (8) comprises means for measuring the power supplied in each moment during the execution of the movement.

This measure allows to define a profile of time variation of the power supplied by the device during the execution of each movement.

In this case, the intensity adjusting cycle can be conveniently configured so that the intensity of each of said light sources (11) oscillates cyclically between a base value and a maximum value, and said base value is obtained as the sum of a minimum threshold value and a value proportional in each moment to the power provided as contribution to the movement by the patient.

The power provided by the patient can be calculated as the difference between the value of power supplied by the mechanism in each moment of a movement cycle in which the patient remains still with his own muscles and the power supplied by the mechanism in the same moment as the current cycle. Such difference indicates in fact the muscle effort applied by the patient.

In this manner, a feedback physical stimulation is provided to the patient, indicating in each moment: the muscle groups in contraction step; the level of applied muscle effort.

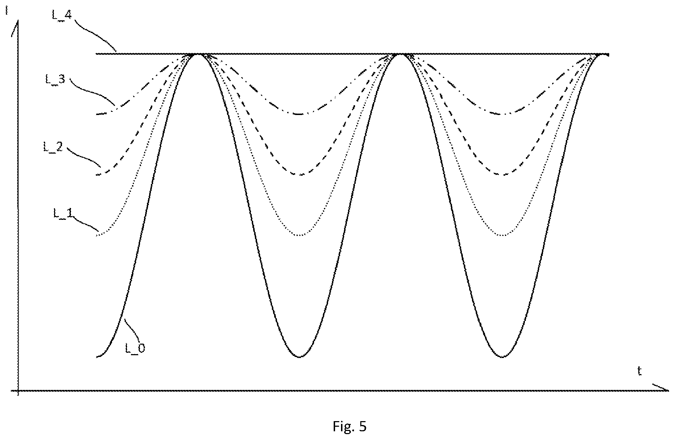

An example of such intensity adjusting cycle, for various effort levels applied by the patient, is shown in the appended figure 5.

At a first effort intensity level (L_0) in which the patient does not contribute in any way to the movement, the intensity (I) of each light source is adjusted so that it varies cyclically, during the movement, between the minimum threshold value and the maximum value, and is synchronized so that the maximum value coincides with the contraction moment of the respective muscle group.

While the effort of the patient increases (level L_1), the base value results from the sum of the minimum threshold value with the value proportional to the muscle effort exerted by the patient, and so it is higher than the base value in case of absence of effort of the patient (L_0).

At another and progressive increase of the effort applied by the patient (L_2, L_3), such base vale increases consequently, up to reach the condition (L_4) in which the patient is able to carry out the movement only relying on his own forces, without the need of an intervention of the machine, and in this case the light radiation intensity is constant and equal to the maximum value.

Preferably, said areas defined at point (1) comprise, for each side of the patient, an area associated to the gluteus, an area associated to the front femoral portion, an area associated to the rear femoral portion, an area associated to the front tibial portion, an area associated to the rear tibial portion.

The couple of lower limbs mobilization actuators (8) can be of passive type.

A display (12), which can be seen by the patient and which reproduces the movement of the patient, can be installed on the base (1).

This synchrony between the thermal stimulations and the walking cycle amplifies the perception the user has of the movement he is carrying out, and speeds up the rehabilitation cycle. Moreover, mild heat is well tolerated and makes the therapy more comfortable for the patient.

In another embodiment the light radiation intensity can be function of the position of said platforms (6), since the position of said platforms indicates the force applied by the patient to compress the pushing elastic means associated to each platform. It is to be specified also that said platforms (6), preferably but not limitingly, can be provided with automatic movement means configured to make the patient's foot carry out movement cycles which simulate the walking movement, and which comprise a series of extensions and flexions of the foot.

Moreover, the movement means of said platforms are preferably provided with measuring means of the mechanical power supplied in order to make the patient's foot carry out the just described movement.

In such case, the device according to the invention can be configured so that the light sources intensity oscillates cyclically between a base value and a maximum value, and said base value is obtained as the sum of a minimum threshold value and a value proportional in each moment to the power provided as contribution to the movement by the patient and calculated as the difference between the value of power supplied by the movement means of the platforms in a movement cycle in which the patient remains still and the power supplied by the same movements means in the same moment as the current cycle. Such difference is in fact indicative of the muscle effort applied by the patient.

In a preferred embodiment the device according to the invention is configured to carry out the following method:

### (100) HEATING OF THE PATIENT

Before carrying out the therapy by means of the machine, the patient is subjected to heating for a time interval between 2 and 5 minutes by means of the provided infrared light sources, in order to produce a general dilation of the blood vessels, with consequent increase of the blood circulation and activation of the metabolism of tissues and cells.

So, by favouring the oxygenation of the muscles and their elasticity, the heating allows to avoid contractures or strains, above all in patients whose limb subjected to the therapy has lost its own functionality. The heat emitted by the infrared rays heats the muscle, thus having a lenitive action which reduces the stress and renders the joints more flexible.

### (200) MOBILIZATION OF LOWER LIMBS AND CONTEMPORARY HEATING OF THE PATIENT

During this step, the intensity of the infrared rays is adjusted so to follow proportionally the intensity of the effort of each muscle group, according to what above described.

While increasing proportionally with the effort of the patient, the heat stimulates the thermoreceptors provided on the skin. Thermoreceptors are receptors sensible to heat and cold which are different from the other sensorial receptors on the skin since they are always active, so to inform the brain constantly about the temperature variation during a cycle imposed by the machine.

By visually observing and perceiving the intensity variation of the infrared rays as a function of the effort made during the movement, the patient will be able to evaluate by himself instantly the success of the rehabilitation.

This will allow the patient to control autonomously his own activity, thus being more involved in the therapy and thus being motivated for improvement.

In this way the patient can interact with the intensity of irradiation according to the effort made, by connecting sensorially to the machine, maximizing the biofeedback.

As yet said, the intensity of the radiation will be proportional to the effort applied by the patient, calculated as the difference between the power needed to move the patient's legs with no collaboration of the same (measured in an initialization cycle of the treatment) and the power supplied during each following cycle.

Such parameter is used to check that the patient works during rehabilitation or training.

In fact, the less power is supplied by the device, the higher the effort will be that the patient applies to carry out the movement.

This makes the power supplied a further instrument able to stimulate the patient: by decreasing such power, the patient has necessarily to participate actively, thus increasing the work he has to carry out.

When the conduction force is deactivated, the movement will be exerted only by the patient able to keep a model of physiological model.

## Claims

1. Device for mobility rehabilitation comprising a table (2), hinged at an end to a support structure (3) and respective actuator (4), configured to make said table (2) rotate between a horizontal position and a vertical position, said table (2) being provided with a harness (15) configured to fasten a patient positioned thereon so that the patient cannot fall down even when the table is in vertical position,
said rehabilitation device comprising also a lower limbs movement mechanism (8) configured to make the patient's femur rotate with respect to the hip and a couple of feet movement platforms (6), **characterized in that**
said device comprises also a plurality of infrared light sources (11), fastened to respective supports (10) integral to said support structure (3) and positioned so that each light source lights up the muscles of a respective area of the patient's lower limbs,
a data processing and control unit (13) configured to control said lower limbs movement mechanism (8) and said light sources (11),
and **in that** said data processing and control unit (13) is configured also to manage an adjusting cycle of the intensity of said light sources (11) in a differentiated manner for each of said sources, as a function of the movement cycle imposed by said couple of mobilization actuators of the lower limbs.

2. Device according to claim 1, **characterized in that** on said processing and control unit (13) computer programs are stored configured to implement the following method:
1) subdividing the lower limbs in a plurality of areas, each infrared light source (11) being configured to stimulate a respective area;
2) for each of said areas defined at point 1, identifying a main muscle group and schematizing the action cycle imposed by said lower limbs mobilization movement mechanism (8) as a sequence of a plurality of contraction and relaxation steps of said main muscle group;
3) determining, for each of said light sources (11) associated to said areas, an adjusting cycle of the intensity of the infrared radiation emitted as a function of said contraction and relaxation cycle schematized at point (2) for the area associated to the specific light source.

3. Device according to claim 2, **characterized in that** the adjusting cycle of the intensity is configured so that each light source has a maximum emission intensity at the contraction steps of the respective muscle group and a minimum emission intensity at the relaxation steps of the respective muscle group.

4. Device according to claim 3, **characterized in that** said movement mechanism (8) comprises means for measuring the power supplied in each moment during the execution of the movement.

5. Device according to claim 4, **characterized in that** said control means are configured to calculate the power provided by the patient as the difference between the value of power supplied by the mechanism in any moment of a movement cycle in which the patient remains still and the power supplied by the mechanism in the same moment as the current cycle.

6. Device according to claim 5, **characterized in that** the intensity adjusting cycle can be configured so that the intensity of each of said light sources (11) oscillates cyclically between a base value and a maximum value, said base value being obtained as the sum of a minimum threshold value and a value proportional in each moment to the power provided as contribution to the movement by the patient.

7. Device according to any one of the preceding claims, **characterized in that** said areas defined at point (1) comprise, for each side of the patient, an area associated to the gluteus, an area associated to the front femoral portion, an area associated to the rear femoral portion, an area associated to the front tibial portion, an area associated to the rear tibial portion.

8. Device according to any one of the preceding claims, **characterized in that** said support structure (3) in integral to a base (1) on rollers.

9. Device according to any one of the preceding claims, **characterized in that** said table is configured to take stably any position between the horizontal and the vertical one.

10. Device according to any one of the preceding claims, **characterized in that** said actuator (4) is a hydraulic or electric piston hinged at an end to the base (1), and at the other end to the table (2) .

11. Device according to any one of the preceding claims, **characterized in that** said movement mechanism comprises a couple of linear actuators, of electric or hydraulic type, each hinged at an end to the table (2) at the position of the hip of each leg of the patient and at the opposite end to a respective fastening means (9) for the patient's femur.

12. Device according to any one of the preceding claims, **characterized in that** said platforms (6) are hinged each with their own front end to the axis of rotation (5) integral to said table (2), and each of said platforms (6) comprises also an elastic pushing means configured to push upwards the rear portion of said platform (6) making it rotate around its own axis of rotation.

13. Device according to any one of claims 1 to 11, **characterized in that** said platforms (6) are hinged each with their own front end to an axis of rotation (5) fastened so that it can translate according to a direction parallel to said table (2), and each of said platforms (6) comprises also an elastic pushing means configured to push upwards the rear portion of said platform (6) making it rotate around its own axis of rotation.

14. Device according to any one of the preceding claims, **characterized in that** the intensity of the light radiation of at least one of said sources is a function of the position of said platforms (6).

15. Device according to any one of the preceding claims, further comprising automatic movement means of said platforms, configured to make the patient's foot carry out movement cycles which simulate the walking movement, and measuring means of the mechanical power supplied by said platform movement means, and **characterized in that** the light sources intensity of at least one of said light sources oscillates cyclically between a base value and a maximum value, said value being obtained as the sum of a minimum threshold value and a value proportional to the difference between the value of power supplied by the movement means of the platforms in a movement cycle in which the patient remains still and the power supplied by the same movements means in the same moment as the current cycle.

## Patentansprüche

1. Vorrichtung zur Mobilitätsrehabilitation, umfassend einen Tisch (2), der an einem Ende an einer Stützstruktur (3) und einem entsprechenden Aktuator (4) angelenkt ist, der so konfiguriert ist, dass der Tisch (2) zwischen einer horizontalen und einer vertikalen Stellung drehen kann, wobei der Tisch (2) mit einem Gurt (15) versehen ist, der so konfiguriert ist, dass ein darauf positionierter Patient so befestigt wird, dass der Patient auch dann nicht herunterfallen kann, wenn sich der Tisch in vertikaler Stellung befindet, wobei das die Rehabilitationsvorrichtung außerdem einen Bewegungsmechanismus (8) für die unteren Gliedmaßen, der so konfiguriert ist, dass der Oberschenkelknochen des Patienten in Bezug auf die Hüfte dreht, und ein Paar Fußbewegungsplattformen (6) umfasst, **dadurch gekennzeichnet,**
**dass** die Vorrichtung außerdem eine Vielzahl von Infrarotlichtquellen (11) umfasst, die an entsprechenden Halterungen (10) befestigt sind, die mit der Stützstruktur (3) integriert und so positioniert sind, dass jede Lichtquelle die Muskeln eines jeweiligen Bereichs der unteren Gliedmaßen des Patienten beleuchtet,
eine Datenverarbeitungs- und Steuereinheit (13), die so konfiguriert ist, dass sie den Bewegungsmechanismus (8) für die unteren Gliedmaßen und die Lichtquellen (11) steuert, und
**dass** die Datenverarbeitungs- und Steuereinheit (13) außerdem so konfiguriert ist, dass sie einen Anpassungszyklus der Intensität der Lichtquellen (11) in differenzierter Weise für jede dieser Quellen als Funktion des Bewegungszyklus verwaltet, der durch das Paar Mobilisierungsaktuatoren der unteren Gliedmaßen auferlegt wird.

2. Vorrichtung nach Anspruch 1, **dadurch gekennzeichnet, dass** auf der Verarbeitungs- und Steuereinheit (13) Computerprogramme gespeichert sind, die so konfiguriert sind, dass sie das folgende Verfahren implementieren:
1) Unterteilen der unteren Gliedmaßen in mehrere Bereiche, wobei jede Infrarotlichtquelle (11) so konfiguriert ist, dass sie einen jeweiligen Bereich stimuliert;
2) für jeden der unter Punkt 1 definierten Bereiche eine Hauptmuskelgruppe identifizieren und den Aktionszyklus, der durch den Mobilisierungsbewegungsmechanismus (8) der unteren Gliedmaßen auferlegt wird, als eine Abfolge mehrerer Kontraktions- und Entspannungsschritte der Hauptmuskelgruppe schematisieren;
3) Bestimmen eines Anpassungszyklus der Intensität der emittierten Infrarotstrahlung für jede der diesen Bereichen zugeordneten Lichtquellen (11) als Funktion des unter Punkt (2) schematisierten Kontraktions- und Entspannungszyklus für den der spezifischen Lichtquelle zugeordneten Bereich.

3. Vorrichtung nach Anspruch 2, **dadurch gekennzeichnet, dass** der Anpassungszyklus der Intensität so konfiguriert ist, dass jede Lichtquelle eine maximale Emissionsintensität bei den Kontraktionsschritten der jeweiligen Muskelgruppe und eine minimale Emissionsintensität bei den Entspannungsschritten der jeweiligen Muskelgruppe aufweist.

4. Vorrichtung nach Anspruch 3, **dadurch gekennzeichnet, dass** der Bewegungsmechanismus (8) Mittel zum Messen der in jedem Moment während der Ausführung der Bewegung gelieferten Leistung umfasst.

5. Vorrichtung nach Anspruch 4, **dadurch gekennzeichnet, dass** die Steuermittel so konfiguriert sind, dass sie die vom Patienten gelieferte Leistung als Differenz zwischen dem Wert der vom Mechanismus in jedem Moment eines Bewegungszyklus gelieferten Leistung, in dem der Patient stillhält, und der vom Mechanismus im selben Moment wie der aktuelle Zyklus gelieferten Leistung berechnen.

6. Vorrichtung nach Anspruch 5, **dadurch gekennzeichnet, dass** der Intensitätsanpassungszyklus so konfiguriert werden kann, dass die Intensität jeder der Lichtquellen (11) zyklisch zwischen einem Basiswert und einem Maximalwert schwankt, wobei der Basiswert als Summe eines minimalen Schwellenwerts und eines Werts erhalten wird, der in jedem Moment proportional zur Leistung ist, die als Beitrag zur Bewegung durch den Patienten bereitgestellt wird.

7. Vorrichtung nach einem der vorhergehenden Ansprüche, **dadurch gekennzeichnet, dass** die an Punkt (1) definierten Bereiche für jede Seite des Patienten einen Bereich umfassen, der dem Gesäßmuskel zugeordnet ist, einen Bereich, der dem vorderen Oberschenkelteil zugeordnet ist, einen Bereich, der dem hinteren Oberschenkelteil zugeordnet ist, einen Bereich, der dem vorderen Schienbeinteil zugeordnet ist, einen Bereich, der dem hinteren Schienbeinteil zugeordnet ist.

8. Vorrichtung nach einem der vorhergehenden Ansprüche, **dadurch gekennzeichnet, dass** die Stützstruktur (3) mit einer Basis (1) auf Rollen integriert ist.

9. Vorrichtung nach einem der vorhergehenden Ansprüche, **dadurch gekennzeichnet, dass** der Tisch so konfiguriert ist, dass er jede Stellung zwischen der Horizontalen und der Vertikalen stabil einnehmen kann.

10. Vorrichtung nach einem der vorhergehenden Ansprüche, **dadurch gekennzeichnet, dass** der Aktuator (4) ein hydraulischer oder elektrischer Kolben ist, der an einem Ende an der Basis (1) und am anderen Ende am Tisch (2) angelenkt ist.

11. Vorrichtung nach einem der vorhergehenden Ansprüche, **dadurch gekennzeichnet, dass** der Bewegungsmechanismus ein Paar linearer Aktuatoren elektrischer oder hydraulischer Art umfasst, die jeweils an einem Ende am Tisch (2) an der Stellung der Hüfte jedes Beins des Patienten angelenkt sind und am gegenüberliegenden Ende an einem jeweiligen Befestigungsmittel (9) für den Oberschenkelknochen des Patienten.

12. Vorrichtung nach einem der vorhergehenden Ansprüche, **dadurch gekennzeichnet, dass** die Plattformen (6) jeweils mit ihrem eigenen vorderen Ende an der Drehachse (5) angelenkt sind, die mit dem Tisch (2) fest verbunden ist, und dass jede der Plattformen (6) auch ein elastisches Schubmittel umfasst, das so konfiguriert ist, dass es den hinteren Teil der Plattform (6) nach oben schiebt, wobei es sie um ihre eigene Drehachse rotieren lässt.

13. Vorrichtung nach einem der Ansprüche 1 bis 11, **dadurch gekennzeichnet, dass** die Plattformen (6) jeweils mit ihrem eigenen vorderen Ende an einer Drehachse (5) angelenkt sind, die so befestigt ist, dass sie sich in einer Richtung parallel zum Tisch (2) verschieben kann, und dass jede der Plattformen (6) außerdem ein elastisches Schubmittel umfasst, das so konfiguriert ist, dass es den hinteren Teil der Plattform (6) nach oben drückt und sie um ihre eigene Drehachse rotieren lässt.

14. Vorrichtung nach einem der vorhergehenden Ansprüche, **dadurch gekennzeichnet, dass** die Intensität der Lichtstrahlung von mindestens einer der Quellen eine Funktion der Stellung der Plattformen (6) ist.

15. Vorrichtung nach einem der vorhergehenden Ansprüche, die außerdem automatische Bewegungsmittel der Plattformen umfasst, die so konfiguriert sind, dass sie den Fuß des Patienten Bewegungszyklen ausführen lassen, die die Gehbewegung simulieren, und Mittel zur Messung der von den Bewegungsmitteln der Plattform gelieferten mechanischen Leistung, und **dadurch gekennzeichnet ist, dass** die Lichtquellenintensität von mindestens einer der Lichtquellen zyklisch zwischen einem Basiswert und einem Maximalwert schwankt, wobei der Wert als Summe eines minimalen Schwellenwerts und eines Werts proportional zur Differenz zwischen dem Wert der von den Bewegungsmitteln der Plattformen in einem Bewegungszyklus gelieferten Leistung, in dem der Patient stillsteht, und der von denselben Bewegungsmitteln im selben Moment wie dem aktuellen Zyklus gelieferten Leistung erhalten wird.

## Revendications

1. Dispositif de rééducation à la mobilité comprenant une table (2), articulée à une extrémité sur une structure de support (3) et un actionneur respectif (4), configuré pour faire tourner ladite table (2) entre une position horizontale et une position verticale, ladite table (2) étant munie d'un harnais (15) configuré pour attacher un patient positionné dessus de manière à ce que le patient ne puisse pas tomber même lorsque la table est en position verticale,
ledit dispositif de rééducation comprenant également un mécanisme de mouvement des membres inférieurs (8) configuré pour faire tourner le fémur du patient par rapport à la hanche et une paire de plates-formes de mouvement des pieds (6), **caractérisé en ce que**
ledit dispositif comprend également une pluralité de sources de lumière infrarouge (11), fixées à des supports respectifs (10) solidaires de ladite structure de support (3) et positionnées de manière à ce que chaque source de lumière éclaire les muscles d'une zone respective des membres inférieurs du patient,
une unité de traitement et de commande de données (13) configurée pour contrôler ledit mécanisme de mouvement des membres inférieurs (8) et lesdites sources de lumière (11),
et **en ce que** ladite unité de traitement de données et de contrôle (13) est configurée également pour gérer un cycle de réglage de l'intensité desdites sources lumineuses (11) de manière différenciée pour chacune desdites sources, en fonction du cycle de mouvement imposé par ladite paire d'actionneurs de mobilisation des membres inférieurs.

2. Dispositif selon la revendication 1, **caractérisé en ce que** sur ladite unité de traitement et de contrôle (13) sont stockés des programmes informatiques configurés pour mettre en œuvre le procédé suivant:
1) subdiviser les membres inférieurs en une pluralité de zones, chaque source de lumière infrarouge (11) étant configurée pour stimuler une zone respective;
2) pour chacune desdites zones définies au point 1, identifier un groupe musculaire principal et schématiser le cycle d'action imposé par ledit mécanisme de mouvement de mobilisation des membres inférieurs (8) comme une séquence d'une pluralité d'étapes de contraction et de relaxation dudit groupe musculaire principal;
3) déterminer, pour chacune desdites sources lumineuses (11) associées auxdites zones, un cycle d'ajustement de l'intensité du rayonnement infrarouge émis en fonction dudit cycle de contraction et de relaxation schématisé au point (2) pour la zone associée à la source lumineuse spécifique.

3. Dispositif selon la revendication 2, **caractérisé en ce que** le cycle de réglage de l'intensité est configuré pour que chaque source lumineuse présente une intensité d'émission maximale aux étapes de contraction du groupe musculaire respectif et une intensité d'émission minimale aux étapes de relaxation du groupe musculaire respectif.

4. Dispositif selon la revendication 3, **caractérisé en ce que** ledit mécanisme de mouvement (8) comprend des moyens pour mesurer la puissance fournie à chaque instant lors de l'exécution du mouvement.

5. Dispositif selon la revendication 4, **caractérisé en ce que** lesdits moyens de commande sont configurés pour calculer la puissance fournie par le patient comme la différence entre la valeur de la puissance fournie par le mécanisme à tout moment d'un cycle de mouvement dans lequel le patient reste immobile et la puissance fournie par le mécanisme au même moment que le cycle en cours.

6. Dispositif selon la revendication 5, **caractérisé en ce que** le cycle de réglage de l'intensité peut être configuré pour que l'intensité de chacune desdites sources lumineuses (11) oscille cycliquement entre une valeur de base et une valeur maximale, ladite valeur de base étant obtenue comme la somme d'une valeur seuil minimale et d'une valeur proportionnelle à chaque instant à la puissance fournie en contribution au mouvement par le patient.

7. Dispositif selon l'une quelconque des revendications précédentes, **caractérisé en ce que** lesdites zones définies au point (1) comprennent, pour chaque côté du patient, une zone associée au fessier, une zone associée à la partie fémorale avant, une zone associée à la partie fémorale arrière, une zone associée à la partie tibiale avant, une zone associée à la partie tibiale arrière.

8. Dispositif selon l'une quelconque des revendications précédentes, **caractérisé en ce que** ladite structure de support (3) est solidaire d'un socle (1) sur roulettes.

9. Dispositif selon l'une quelconque des revendications précédentes, **caractérisé en ce que** ladite table est configurée pour prendre de manière stable n'importe quelle position entre l'horizontale et la verticale.

10. Dispositif selon l'une quelconque des revendications précédentes, **caractérisé en ce que** ledit actionneur (4) est un piston hydraulique ou électrique articulé à une extrémité au socle (1), et à l'autre extrémité à la table (2).

11. Dispositif selon l'une quelconque des revendications précédentes, **caractérisé en ce que** ledit mécanisme de mouvement comprend un couple d'actionneurs linéaires, de type électrique ou hydraulique, chacun articulé à une extrémité de la table (2) à la position de la hanche de chacune jambe du patient et à l'extrémité opposée à un moyen de fixation respectif (9) pour le fémur du patient.

12. Dispositif selon l'une quelconque des revendications précédentes, **caractérisé en ce que** lesdites plateformes (6) sont articulées chacune avec leur propre extrémité avant à l'axe de rotation (5) solidaire de ladite table (2), et chacune desdites plateformes (6) comprend également un moyen de poussée élastique configuré pour pousser vers le haut la partie arrière de ladite plateforme (6) en la faisant tourner autour de son propre axe de rotation.

13. Dispositif selon l'une quelconque des revendications 1 à 11, **caractérisé en ce que** lesdites plateformes (6) sont articulées chacune avec leur propre extrémité avant à un axe de rotation (5) solidaire de manière à pouvoir se déplacer selon une direction parallèle audit table (2), et chacune desdites plateformes (6) comprend également un moyen de poussée élastique configuré pour pousser vers le haut la partie arrière de ladite plateforme (6) en la faisant tourner autour de son propre axe de rotation.

14. Dispositif selon l'une quelconque des revendications précédentes, **caractérisé en ce que** l'intensité du rayonnement lumineux d'au moins une desdites sources est une fonction de la position desdites plateformes (6).

15. Dispositif selon l'une quelconque des revendications précédentes, comprenant en outre des moyens de déplacement automatique desdites plateformes, configurés pour faire effectuer au pied du patient des cycles de mouvements simulant le mouvement de marche, et des moyens de mesure de la puissance mécanique fournie par lesdits moyens de déplacement de la plateforme, et **caractérisé en ce que** l'intensité des sources lumineuses d'au moins une desdites sources lumineuses oscille cycliquement entre une valeur de base et une valeur maximale, ladite valeur étant obtenue comme la somme d'une valeur seuil minimale et d'une valeur proportionnelle à la différence entre la valeur de puissance fournie par les moyens de mouvement des plateformes dans un cycle de mouvement dans lequel le patient reste immobile et la puissance fournie par les mêmes moyens de mouvement au même moment que le cycle en cours.
